# EUROPEAN PATENT APPLICATION

(11) **EP 2 933 339 A1**
(43) Date of publication of application: **21.10.2015**
(21) Application number: 13861721.2
(22) Date of filing: 04.12.2013
(51) Int. Cl.: C12P 7/18, C12N 1/19, C12N 1/21

(54) **METHOD FOR MANUFACTURING BUTANEDIOLS, METHOD FOR PRODUCTION OF MICROORGANISMS FOR MANUFACTURING BUTANEDIOLS, AND MICROORGANISMS**

(30) Priority: 12.12.2012 JP 2012271519
(71) Applicant: Showa Denko K.K., Tokyo 105-8518 (JP)
(72) Inventor: AOKI, Hirobumi, Tokyo 105-8518 (JP); KOKIDO, Yuzuru, Tokyo 105-8518 (JP); HASHIMOTO, Yoko, Tokyo 105-8518 (JP); YONEDA, Tadashi, Tokyo 105-8518 (JP)
(74) Representative: Strehl Schübel-Hopf & Partner
(86) International application number: PCT/JP2013/082634
(87) International publication number: WO 2014/091991

(57) **Abstract**

A method of manufacturing a butanediol through 3-hydroxybutyryl-CoA that uses a microbe and/or a culture thereof and utilizes an enzyme reaction that is caused by an acyl-CoA reductase, wherein the microbe in the manufacturing method for a butanediol is characterized in that an activity of an acyl-CoA hydratase (EC number: 3. 1. 2. -) is deleted or reduced.

## Description

### TECHNICAL FIELD

The present invention relates to a manufacturing method for a butanediol, a fabrication method for a microbe for manufacturing a butanediol, and a microbe.

### BACKGROUND ART

In recent years, attention is paid to a compound manufacturing process with a renewable source as a raw material from the viewpoint of depletion of fossil resource, a countermeasure for global warming, or the like. In particular, a so-called bio-refinery has widely been studied wherein a variety of compounds as raw materials for a polymer or compounds as raw materials for a chemical product are manufactured in biochemical processes with biomass as raw materials.

For a compound that is expected for raw material conversion of biomass, a butanediol is provided. For example, 1,3-butanediol as a solvent and 1,4-butanediol as a synthetic raw material for a precision organic chemical product, monomer units of a polyester and an engineering plastic, or the like are widely used, and a market size thereof is large. For that reason, demand for a method of manufacturing a butanediol efficiently in a biochemical process with a renewable source such as biomass as a raw material is increased.

For manufacturing methods for a butanediol that use a biochemical process, there are provided, for example, methods described in patent documents 1 and 2 and non-patent document 1.

### PRIOR ART DOCUMENTS

[Patent Document 1] Japanese Patent No. 4380704 specification
[Patent Document 2] International Publication No. 2008/115840 official gazette

[Non-patent document 1] Harry Yim et al., Metabolic engineering of Escherichia coli for direct production of 1,4-butanediol, Nature Chemical Biology, 7, 445-452 (2011).

### DISCLOSURE OF THE INVENTION

### PROBLEMS TO BE SOLVED BY THE INVENTION

However, methods described in patent documents 1 and 2 and non-patent document 1 are complicated processes.

Against a problem as described above, an economic manufacturing method for 1,4-butanediol is provided.

### MEANS FOR SOLVING THE PROBLEM

The present invention includes the following.
[1] A method of manufacturing a butanediol through 3-hydroxybutyryl-CoA that uses a microbe and/or a culture thereof and utilizes an enzyme reaction that is caused by an acyl-CoA reductase, wherein the microbe in the manufacturing method for a butanediol is characterized in that an activity of an acyl-CoA hydratase (EC number: 3. 1. 2. -) is deleted or reduced.
[2] The manufacturing method for a butanediol as described in [1], wherein the acyl-CoA hydratase is an acyl-CoA hydratase classified into EC number: 3. 1. 2. 2 or EC number: 3. 1. 2. 20.
[3] The manufacturing method for a butanediol as described in [1], wherein the microbe includes a gene that codes β-ketothiolase (EC number: 2. 3. 1. 9), a gene that codes 3-hydroxybutyryl-CoA dehydrogenase (EC number: 1. 1. 1. 35), and a gene that codes acyl-CoA reductase (EC number: 1. 2. 1. 10).
[4] The manufacturing method for a butanediol as described in [3], wherein the microbe further includes a gene that codes enoyl-CoA hydratase (EC number: 4. 2. 1. 17), a gene that codes vinylacetyl-CoA delta-isomerase (EC number: 5. 3. 3. 3 ), and a gene that codes 4-hydroxybutyryl-CoA dehydratase (EC number: 4. 2. 1. 120).
[5] The manufacturing method for a butanediol as described in [1], wherein the microbe is Escherichia coli, a yeast, a coryneform bacteria, or a clostridial bacteria.
[6] The manufacturing method for a butanediol as described in [5], wherein the microbe is Escherichia coli with deleted CDS region of acyl-CoA thioesterase II gene (tesB).
[7] A fabrication method for a microbe for manufacturing a butanediol, characterized by including a process for deleting or reducing an activity of an acyl-CoA hydratase (EC number: 3. 1. 2. -) of a microbe.
[8] The fabrication method for a microbe for manufacturing a butanediol as described in [7], wherein the acyl-CoA hydratase is an acyl-CoA hydratase classified into EC number: 3. 1. 2. 2 or EC number: 3. 1. 2. 20.
[9] A microbe that includes a gene that codes β-ketothiolase (EC number: 2. 3. 1. 9), a gene that codes 3-hydroxybutyryl-CoA dehydrogenase (EC number: 1. 1. 1. 35), and a gene that codes acyl-CoA reductase (EC number: 1. 2. 1. 10) and a deleted or reduced activity of an acyl-CoA hydratase (EC number: 3. 1. 2. -).
[10] The microbe as described in [9], wherein the acyl-CoA hydratase is an acyl-CoA hydratase classified into EC number: 3. 1. 2. 2 or EC number: 3. 1. 2. 20.
[11] The microbe as described in [9], further including a gene that codes enoyl-CoA hydratase (EC number: 4. 2. 1. 17), a gene that codes vinylacetyl-CoA delta-isomerase (EC number: 5. 3. 3. 3), and a gene that codes 4-hydroxybutyryl-CoA dehydratase (EC number: 4. 2. 1. 120).
[12] The microbe as described in [9], wherein the microbe is Escherichia coli, a yeast, a coryneform bacteria, or a clostridial bacteria.

### EFFECTS OF THE INVENTION

It is possible to provide a selective or economic manufacturing method for a butanediol.

### BRIEF DESCRIPTION OF THE DRAWINGS

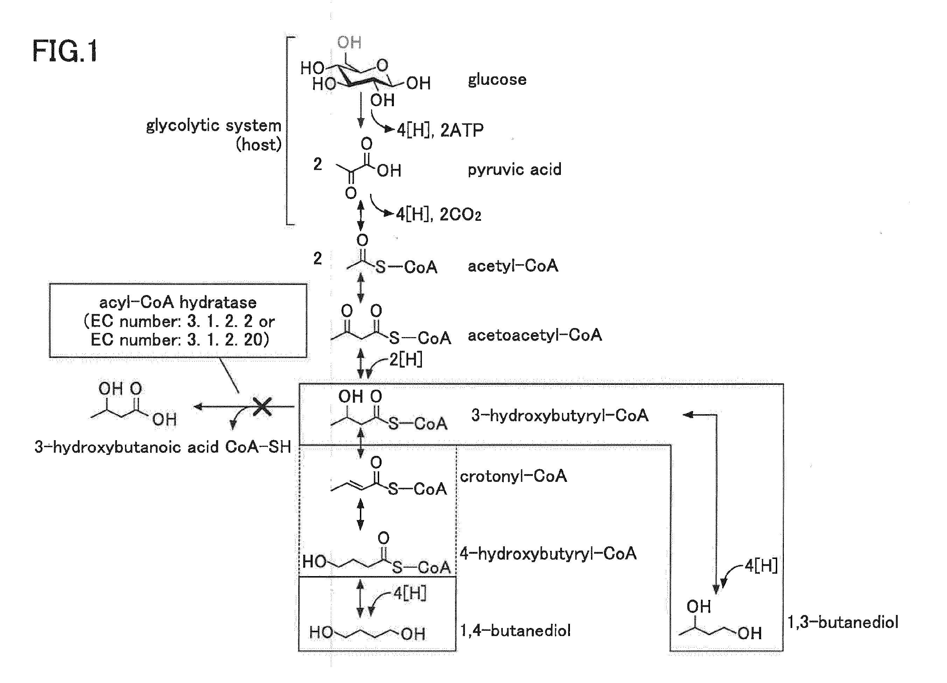

[FIG. 1] FIG. 1 is one example of an enzymatic system of a manufacturing method for a butanediol according to the present embodiment.

### BEST MODE FOR IMPLEMENTING THE INVENTION

The present invention will be described in detail below. Here, "CoA" in the present specification means "coenzyme A". Furthermore, "%" indicates "% by mass" unless otherwise described. "ppm" is a mass standard.

The present embodiment includes a manufacturing method for a butanediol through 3-hydroxybutyryl-CoA that uses a microbe and includes a reduction process that is caused by an acyl-CoA reductase. The inventors variously studied in order to improve productivity of a butanediol. Specifically, presence of a (side reaction) enzyme system was investigated in detail that was intrinsically possessed by a host microbe and greatly influenced productivity of a butanediol. As a result, it was found that a reaction of production of 3-hydroxybutanoic acid from 3-hydroxybutyryl-CoA that is caused by an enzyme originating from a host was one of side reaction enzyme systems. That is, it was found that a reaction of hydrolysis from 3-hydroxybutyryl-CoA into 3-hydroxybutanoic acid influenced a reduction process caused by an acyl-CoA reductase greatly, that is, production of a butanediol ultimately.

Based on such a finding, the inventors further found that it was possible to suppress a reaction of hydrolysis from 3-hydroxybutyryl-CoA into 3-hydroxybutanoic acid by deleting or reducing an activity of a particular acyl-CoA hydratase (EC number: 3. 1. 2. -) that was used in such a biochemical manufacturing method and possessed by a host bacteria, and as a result, it was possible to increase an amount of a produced butanediol.

A feature of a microbe, a fabrication method for a microbe, a using method for a microbe (that is, a manufacturing method for a butanediol), an acquisition method for a manufactured butanediol, and the like, that are used in the present embodiment, will be described below.

### (A host microbe)

A host microbe that is used in the present embodiment is a microbe with a deleted or reduced activity of an acyl-CoA hydratase. As such a microbe is used as a host for manufacturing a butanediol, hydrolysis of 3-hydroxybutyryl-CoA due to an acyl-CoA hydratase to derive 3-hydroxybutanoic acid is suppressed in a manufacturing method for a butanediol through 3-hydroxybutyryl-CoA that includes a reduction process that is caused by an acyl-CoA reductase, and as a result, productivity of a butanediol is improved.

An "acyl-CoA hydratase" that is a target for deleting or reducing an activity thereof is not particularly limited as long as such an enzyme has an activity of decomposition of 3-hydroxybutyryl-CoA, and acyl-CoA hydratase that refers to EC number: 3. 1. 2. 2, acyl-CoA hydratase that refers to EC number: 3. 1. 2. 20, and homologs thereof, and the like are provided specifically. These are also commonly referred to as acyl-CoA thioesterase, thioesterase, thioesterase II, and the like, and an "acyl-CoA hydratase" in the present invention encompasses them.

Here, "deletion or reduction" of an activity in the present specification means that an activity of an acyl-CoA hydratase as a target is reduced into a half or less of that of a parent strain that is a target of such a modification. Due to such deletion or reduction, decomposition of 3-hydroxybutyryl-CoA is avoided and an amount of a produced butanediol is increased substantially. Here, in any embodiment that uses a bacterial strain, it is preferable for an activity of an "acyl-CoA hydratase" to be reduced a half or less as compared with that of Escherichia coli JM109 strain. It is possible to confirm "deletion or reduction" in the present embodiment as follows. For example, such a host microbe is transformed with a gene that codes a series of enzymes for producing 3-hydroxybutyryl-CoA, and cultured in a culture medium that contains a carbon source that is a substrate for such an enzyme reaction, for example, glucose or the like. Produced 3-hydroxybutyryl-CoA is hydrolyzed under presence of an acyl-CoA hydratase and excreted as 3-hydroxybutanoic acid out of a bacterial body. Such 3-hydroxybutanoic acid is quantified by a publicly known method such as a high performance liquid chromatography (HPLC), and thereby, it is possible to evaluate "deletion or reduction" of an acyl-CoA hydratase.

An example of a microbe that is used in the present embodiment and is a parent strain as a target for modification is a host microbe wherein it is possible to introduce therein a variety of genes as described below, and such a microbe is not particularly limited as long as it is possible to apply a gene recombination technique thereto and express an acyl-CoA hydratase that is a target for deletion or reduction of an activity thereof.

For a specific example of a host microbe usable in the present embodiment, an Escherichia coli, a yeast, a coryneform bacteria, or a clostridial bacteria is provided from the viewpoint of industrial availability. For a yeast, there is provided Saccharomyces cerevisiae, Schizosaccharomyces pombe, Kluyveromyces lactis, Kluyveromyces marxianus, or the like. For a coryneform bacteria, there is provided Corynebacterium glutamicum, Corynebacterium efficiens, Brevibacterium divaricatum, Brevibacterium saccharolyticum, Brevibacterium immariophilum, Brevibacterium lactofermentum, Brevibacterium roseum, Brevibacterium flavum, Brevibacterium thiogenitalis, Corynebacterium acetoacidophilum, Corynebacterium acetoglutamicum, Corynebacterium callunae, Corynebacterium lilium, Corynebacterium mellassecola, Microbacterium ammoniaphilum, or the like. For a clostridial bacteria, there is provided Clostridium kluyveri, Clostridium acetobutylicum, Clostridium aminobutyricum, Clostridium beijerinckii, Clostridium saccharoperbutylacetonicum, or the like. Among these, it is preferable to use an Escherichia coli, Saccharomyces cerevisiae, Schizosaccharomyces pombe, or Corynebacterium glutamicum, because transformation thereof is easy, and it is more preferable to use an Escherichia coli.

For an Escherichia coli, there are provided, for example, an Escherichia coli K-12 strain or B strain and derivatives thereof, more specifically, Escherichia coli BW strain such as BW25113 strain, JM strain such as JM109 strain, MV strain such as MV1184 strain, DH strain such as DH5α, HB101 strain, C600 strain, XL1-Blue strain, BL21 strain, and the like, and derivatives thereof.

### [Fabrication of a host microbe with a deleted or reduced activity of an acyl-CoA hydratase]

A method for fabrication of a host with a deleted or reduced activity of an acyl-CoA hydratase that is used in the present embodiment is not particularly limited and it is possible to execute fabrication by a known method for deleting or reducing expression of a gene that is possessed by a microbe. For a specific example thereof, there is provided chemical modification that uses N-methyl-N'-nitro-N-nitrosoguanidine (NTG), ethyl methanesulfonate (EMS), or the like, induction of an error at a time of replication that uses an intercalation agent, introduction of random mutation into DNA that uses error induction or the like in a response to repair of a physical damage that uses an ultraviolet ray, a radiation ray, or the like, a method based on deletion or insertion of DNA induced by a transposon, phage infection, or the like, or the like. Furthermore, in a case where a base sequence of an enzyme that is a target is known, a method of introduction of deletion or insertion into DNA that utilizes homologous recombination caused by introducing a DNA fragment that has a complementary sequence with respect to a gene thereof or a marginal region thereof, or the like, may be adopted. For confirmation of introduction of such mutation or an effect thereof, it is possible to apply a variety of known DNA sequence analysis techniques, and additionally, it is possible to execute confirmation by analyzing substantial reduction of such an enzyme reaction product that is caused by deletion of such an enzyme.

It is possible to execute introduction of a gene into a host microbe by appropriately combining and using a variety of known methods, for example, a method based on a restriction enzyme / ligation, an In-Fusion cloning method, and the like, so that a gene as described above or a part thereof is linked with an appropriate vector and an obtained recombinant vector is introduced into a host in such a manner that it is possible for a target gene to be expressed. Alternatively, it is possible to insert a target gene or a part thereof at an arbitrary position on genome by means of homologous recombination. A "part" refers to a part of each gene that is capable of expressing a protein that is coded by each gene in a case of being introduced into a host. A gene in the present invention encompasses DNA and RNA, and preferably, is DNA.

A vector that is linked with a gene as described previously is not particularly limited as long as it is possible to execute replication thereof in a host, and there are provided, for example, a plasmid, a phage, a cosmid, and the like, that are utilized for introduction of an exotic gene into Escherichia coli. For a plasmid, there is provided, for example, pHSG398, pUC18, pBR322, pSC101, pUC19, pUC118, pUC119, pACYC117, pBluescript II SK(+), pET17b, pETDuet-1, pACYCDuet-1, pCDFDuet-1, pRSFDuet-1, pCOLADuet-1, or the like, and for a phage, there is provided, for example, λgt10, Charon 4A, EMBL-, M13mp18, M13mp19, or the like. Some of them are commercially available and it is possible to use a commercially available product (kit) directly in accordance with, or by appropriately modifying, a procedure manual thereof.

In a vector as described above, an appropriate expression promoter may be connected to an inserted gene upstream thereof so that such a gene is expressed reliably. An expression promoter to be used is not particularly limited, and it is possible for a person skilled in the art to make an appropriate selection thereof depending on a host. For example, it is also possible to utilize T7 promoter, lac promoter, trp promoter, trc promoter, or λ-PL promoter that is utilized for expression of an exotic gene in Escherichia coli, or an Nar promoter region of a nitrate reduction gene nar GHJI operon that is derived from Escherichia coli and involved with nitrate respiration, or a promoter region of Frd gene that is a gene of a nitrate reductase of Escherichia coli.

Furthermore, depending on a case, it is also preferable to destruct an intrinsic gene of a host microbe so that such a gene is not expressed. For a method of gene destruction, it is possible to use a publicly-known method that is utilized for gene destruction in Escherichia coli. Specifically, it is possible to use a method that is used for fabricating a knockout cell or the like in such a technical field, such as a method (gene targeting method) that destructs a target gene by using a vector (targeting vector) that causes homologous recombination at an arbitrary position of such a gene, a method (gene trap method) that inserts a trap vector (reporter gene that does not have a promoter) at an arbitrary position of a target gene to destruct such a gene and be caused to lose a function thereof, or a method of a combination thereof. Furthermore, it is also possible to utilize a method that introduces a vector that expresses an antisense cDNA of a gene to be destructed or a method that introduces into a cell a vector that expresses a vector that expresses a double-stranded RNA of a gene to be destructed. Such a vector encompasses a virus vector, a plasmid vector, or the like, it is possible to execute fabrication thereof based on a normal gene engineering technique, for example, in accordance with a basic literature such as Sambrook, J et al., Molecular Cloning 2nd ed., 9.47 - 9.58, Cold Spring Harbor Lab. press (1989). Furthermore, it is also possible to cleave a commercially available vector with an arbitrary restriction enzyme and incorporate a desired gene or the like to execute semisynthesis.

A position that causes homologous substitution or a position for inserting a trap vector is not particularly limited as long as such a position is to cause mutation that eliminates expression of a target gene to be destructed, and is preferably a transcription control region.

Moreover, a method for introduction of a vector as described previously into a host is not particularly limited, and it is possible to provide, for example, an electrical pulse method, a method that uses a calcium ion, a protoplast method, an electroporation method, or the like, that is generally utilized for introduction of a vector into Escherichia coli.

A target gene together with a promoter is inserted into a sequence homologous to a sequence on a genome and such a nucleic acid fragment is introduced into a cell by means of electroporation to cause homologous recombination, so that it is possible to practice a method that inserts a target gene at an arbitrary position on a genome by means of homologous recombination. As a nucleic acid fragment wherein a drug resistance gene is linked with a target gene is used in a case of introduction into a genome, it is possible to readily choose a strain wherein homologous recombination is caused. Furthermore, it is also possible to introduce a target gene by means of homologous recombination in such a manner that a gene wherein a gene that is lethal on a particular condition is linked with a drug resistance gene is inserted into a genome by means of homologous recombination in a method as described above and subsequently the drug resistance gene and the gene that is lethal on a particular condition are replaced.

Moreover, a method that selects a recombinant microbe with an introduced target gene is not particularly limited, and it is preferable to be based on a technique that is capable of readily selecting only a recombinant microbe with an introduced target gene.

Here, a transformed microbe in the present embodiment may be used as a microbe culture bacterial body itself or a variety of forms of a culture thereof. Specifically, a culture of a microbe in the present embodiment includes a suspension of a microbe culture bacterial body in a medium such as a culture medium or a buffer solution, a cell-free extracted fluid from a microbe culture bacterial body, and further, a product processed by concentrating, purifying, and extracting a component that catalyzes such a reaction from such a cell-free extracted fluid or the like. A culture of a microbe in the present embodiment further includes the above-mentioned processed product of a microbe that is fixed on a poorly soluble carrier. For such a fixation carrier, there is provided a compound that forms a poorly watersoluble solid content that encloses a microbial or bacterial body as described previously or a processed product thereof, such as polyacrylamide, polyvinyl alcohol, poly-N-vinylformamide, polyallylamine, polyethyleneimine, methylcellulose, glucomannan, alginate, or carrageenan, or further a copolymer or crosslinked product thereof, or the like. One kind of these may be used singly or two or more kinds thereof may be mixed and used. Furthermore, it is also possible to use, as a culture of a microbe, a microbe or an extracted fluid or extracted component thereof that is held on an object that is preliminarily formed as a solid, such as activated carbon, a porous ceramic, glass fiber, a porous polymer molded object, or a nitrocellulose film.

### (Manufacturing of a butanediol)

FIG. 1 illustrates one example of an enzyme system in a manufacturing method for a butanediol (1,3-butanediol and 1,4-butanediol) according to the present embodiment. In the present embodiment, it is possible to obtain a butanediol by using a culture that is expressed in a microbial body by transforming a series of genes as described previously or the like. Here, a gene is inserted into an arbitrary vector individually or as a series of clusters so that a host microbe is transformed. An obtained transformed body is cultured in a culture medium with an appropriate carbon source, for example, glucose as a carbon source, so that each gene is expressed. In a case of a gene capable of being constitutively expressed in a host, a transformed body is cultured in a culture medium so that such a gene is expressed. On the other hand, in a case where each gene is constituted under a control of a regulator disposed on a vector, an inducible substrate is added to transfer to inductive environment, and thereby, each coding gene is expressed. Here, culturing in the present embodiment includes all of culturing conditions for a normal microbe culturing, and further, a culturing step means that a microbe is cultured for a period of time and a condition enough to manufacture a butanediol.

### (Manufacturing of 1,3-butanediol)

An enzyme in each enzyme reaction will be described below.

### [Supply of 3-hydroxybutyryl-CoA]

A method for supply of 3-hydroxybutyryl-CoA in a manufacturing method for 1,3-butanediol in the present embodiment is not particularly limited and a variety of known methods are used.

For one example, acetoacetyl-CoA is first supplied by utilizing an enzyme reaction that uses a gene that codes β-ketothiolase (EC number: 2. 3. 1. 9) that eliminates one molecule of CoA from two molecules of acetyl-CoA, under supply of acetyl-CoA that is obtained from a known reaction path such as a glycolytic system, so as to furnish acetoacetyl-CoA, or a homolog thereof. Furthermore, a gene that codes acetoacetyl-CoA synthase (EC number: 2. 3. 1. 194) that catalyzes a reaction that irreversibly produces acetoacetyl-CoA wherein acetyl-CoA and malonyl-CoA are substrates, or a homolog thereof, may be used to supply acetoacetyl-CoA. For details of acetoacetyl-CoA synthase, it is possible to refer to, for example, a non-patent document such as Unprecedented acetoacetyl-coenzyme A synthesizing enzyme of the thiolase superfamily involved in the mevalonate pathway., Proc. Natl. Acad. Sci., U.S.A. 107, 11265-11270 (2010). Then, it is possible to obtain 3-hydroxybutyryl-CoA through an enzyme reaction that uses acetoacetyl-CoA reductase (EC number: 1. 1. 1. 36), 3-hydroxybutyryl-CoA dehydrogenase (EC number: 1. 1. 1. 35), 3-hydroxyacyl-CoA dehydrogenase (EC number: 1. 1. 1. 157), or a homolog thereof that catalyzes a reaction that reduces obtained acetoacetyl-CoA to furnish 3-hydroxybutyryl-CoA. Here, it is possible to obtain (R)-3-hydroxybutyryl-CoA in a case where acetoacetyl-CoA reductase (EC number: 1. 1. 1. 36) is used, or it is possible to obtain (S)-3-hydroxybutyryl-CoA in a case where 3-hydroxybutyryl-CoA dehydrogenase (EC number: 1. 1. 1. 35) or 3-hydroxyacyl-CoA dehydrogenase (EC number: 1. 1. 1. 157) is used.

Furthermore, for another example, propionic acid-CoA transferase (EC number : 2. 8. 3. 1) is known as an enzyme that directly transforms CoA into 3-hydroxybutanoic acid. It is possible to produce 3-hydroxybutyryl-CoA by supplying 3-hydroxybutanoic acid to a microbe or a culture that includes such a microbe, under expression of a gene that codes propionic acid-CoA transferase or a homolog thereof and under supply of acetyl-CoA that is a donor for a CoA transfer reaction.

### [An acyl-CoA reductase]

An acyl-CoA reductase that is used in the present embodiment is not particularly limited as long as such an enzyme catalyzes a reaction that reduces 3-hydroxybutyryl-CoA to produce 3-hydroxybutanal.

For a specific example of an enzyme that catalyzes a reaction as described above, there is provided aldehyde dehydrogenase (acylation) (EC number: 1. 2. 1. 10) or a homolog thereof.

Here, although 1,3-butanediol is derived from obtained 3-hydroxybutanal by an alcohol reductase that is normally possessed by a host as described below, a gene that codes an alcohol reductase may additionally be expresses to manufacture 1,3-butanediol.

### (Manufacturing of 1,4-butanediol)

FIG. 1 illustrates one example of an enzyme system in a manufacturing method for 1,4-butanediol in the present embodiment. For manufacturing of 1,4-butanediol in accordance with the present embodiment, it is possible to derive 1,4-butanediol from 3-hydroxybutyryl-CoA that is supplied similarly to manufacturing of 1,3-butanediol as described previously, by utilizing, for example, an enzyme reaction through crotonyl-CoA and 3-hydroxybutyryl-CoA. An enzyme in each enzyme reaction will be described below.

### [An enoyl-CoA hydratase]

For a specific example of an enzyme that is used in the present embodiment and catalyzes a reaction that dehydrates (S)-3-hydroxybutyryl-CoA to crotonyl-CoA, there is provided enoyl-CoA hydratase (EC number: 4. 2. 1. 17) or a homolog thereof. For details of an enzyme as described above, it is possible to refer to a non-patent document such as Moskowitz, G. J. and Merrick, J. M. Metabolism of poly-β-hydroxybutyrate. II. Enzymatic synthesis of D-(-)-β-hydroxybutyryl coenzyme A by an enoyl hydrase from Rhodospirillum rubrum. Biochemistry 8 (1969) 2748 - 2755.

Furthermore, a specific example of an enzyme that is used in the present embodiment and catalyzes a reaction that produces crotonyl-CoA from (R)-3-hydroxybutyryl-CoA is not particularly limited and there is provided enoyl-CoA hydratase (EC number: 4. 2. 1. 55 (3-hydroxybutyryl-CoA dehydratase) or EC number: 4. 2. 1. 119), or the like. For details of an enzyme as described above, it is possible to refer to a non-patent document such as Fukui, T., Shiomi, N. and Doi, Y. Expression and characterization of (R)-specific enoyl coenzyme A hydratase involved in polyhydroxyalkanoate biosynthesis by Aeromonas caviae. J. Bacteriol. 180 (1998) 667 - 673 or Metabolism of poly-beta-hydroxybutyrate. II. Enzymatic synthesis of D-(-)-beta hydroxybutyryl coenzyme A by an enoyl hydrase from Rhodospirillum rubrum., Moskowitz GJ, Merrick JM. Journal Biochemistry, 8 2748 - 2755 (1969), or the like.

### [A vinylacetyl-CoA delta-isomerase]

A vinylacetyl-CoA delta-isomerase that is used in the present embodiment is not particularly limited as long as such an enzyme catalyzes a reaction that transforms an olefin of crotonyl-CoA to produce vinylacetyl-CoA.

A specific example of an enzyme that catalyzes a reaction as described above is not limited and there is provided vinylacetyl-CoA delta-isomerase (EC number: 5. 3. 3. 3) or a homolog thereof or the like. For details of an enzyme as described above, it is possible to refer to, for example, a non-patent document such as Fermentation of 4-aminobutyrate by Clostridium aminobutyricum: cloning of two genes involved in the formation and dehydration of 4-hydroxybutyryl-CoA, Archives of Microbiology, 174 (3) 189 - 199 (2000).

### [A 4-hydroxybutyryl-CoA dehydratase]

A 4-hydroxybutyryl-CoA dehydratase is not particularly limited in the present embodiment as long as such an enzyme catalyzes a reaction that hydrates vinylacetyl-CoA to produce 4-hydroxybutyryl-CoA.

For a specific example of an enzyme that catalyzes a reaction as described above, there is provided 4-hydroxybutyryl-CoA dehydratase (EC number: 4. 2. 1. 120) or a homolog thereof, or the like. For details of an enzyme as described above, it is possible to refer to, for example, a non-patent document such as Fermentation of 4-aminobutyrate by Clostridium aminobutyricum: cloning of two genes involved in the formation and dehydration of 4-hydroxybutyryl-CoA, Archives of Microbiology, 174 (3) 189 - 199 (2000). Here, a case in this non-patent document relates to a multienzyme of 4-hydroxybutyryl-CoA dehydratase and vinylacetyl-CoA delta-isomerase (EC number: 5. 3. 3. 3) as described previously, and a gene that codes it. However, as long as it is possible to provide each enzyme function suitably, a gene that codes each enzyme may be used individually or a gene that codes an enzyme protein or a catalytic subunit may be used.

### [An acyl-CoA reductase]

An acyl-CoA reductase that is used in the present embodiment is not particularly limited as long as such an enzyme catalyzes a reaction that reduces 4-hydroxybutyryl-CoA to produce 4-hydroxybutanal.

For a specific example of an enzyme that catalyzes a reaction as described above, there is provided aldehyde dehydrogenase (acylation) (EC number: 4. 2. 1. 10) or a homolog thereof.

Here, although 1,4-butanediol is derived from obtained 4-hydroxybutanal by an alcohol reductase that is normally possessed by a host as described below, a gene that codes an alcohol reductase may additionally be expressed to manufacture 1,4-butanediol.

A homolog In the present embodiment includes an ortholog and a paralog. An ortholog refers to a set of corresponding genes among species generated from a gene of a common ancestor by means of speciation and enzymes obtained from such genes. A paralog refers to corresponding genes among species generated by means of not speciation but gene duplication in an identical species and enzymes obtained from such genes. A homolog refers to genes that have an identity in sequences thereof, regardless of an ortholog or a paralog, and enzymes obtained from such genes.

More specifically, a homolog (gene) of a gene as described above refers to a gene that has a base sequence with an identity greater than or equal to 90%, preferably an identity greater than or equal to 95%, with respect to such a gene, and more preferably, a gene that is completely identical to such a gene or wherein one or several bases thereof are deleted, substituted, or added.

Furthermore, a homolog gene includes a gene that hybridizes with a gene that has a base sequence complementary with a target gene on a stringent condition. Specifically, it is possible to acquire a gene or an enzyme that is obtained by transformation caused by such a gene, by applying a homology retrieval program (for example, BLAST or FASTA) to a publicly-known data base, or based on an ordinary method such as hybridization or polymerase chain reaction (PCR) on a stringent condition that uses a probe that is composed of at least a portion of an identified gene (DNA that is composed of a base sequence complementary with DNA that is composed of a base sequence of such a gene). Furthermore, it is possible for a person(s) skilled in the art to execute self-design by substituting a base sequence or the like. Here, for a stringent condition referred herein, there is provided, for example, a hybridizing condition described in a non-patent document of Molecular Cloning - A LABORATORY MANUAL THIRD EDITION (Joseph Sambrook, David W. Russell., Cold Spring Harbor Laboratory Press). More specifically, a hybridizing condition is a condition that retention with a probe in a solution that contains 6 x SSC (a composition of 1 x SSC: 0.15 M of sodium chloride, 0.015 M of sodium citrate, pH: 7.0), 0.5% of SDS, 5 x Denhardt's solution, and 100 mg/mL of herring sperm DNA, at a constant temperature of 65 °C for 8 - 16 hours is executed to cause hybridization.

### (A culturing method)

For example, a reaction in the present invention is most conveniently achieved in such a manner that a transformed body is cultured in a nutritive culture medium such as an LB culture medium at a temperature of 15 °C - 40 °C, desirably 18 °C - 37 °C for about 24 hours, subsequently implanted to a culture medium with a normal carbon source, for example, a carbon source that is 0.01 - 50%, desirably 0.1 - 30%, of glucose, and continuously cultured at a similar temperature for about 1 hour - 200 hours, wherein 1,3-butanediol and/or 1,4-butanediol is stored in a culture solution in such a process. Furthermore, a carbon source may be added continuously or intermittently depending on consumption of a carbon source that is caused by proliferation of a bacteria or proceeding of a reaction, and in such a case, a concentration of a carbon source in a reaction fluid is not limited to one described previously.

For a culture medium carbon source for culturing a microbe, it is possible to use a saccharide such as glucose, sucrose, or fructose, a polyol such as glycerol, an organic substance such as ethanol, acetic acid, citric acid, succinic acid, lactic acid, benzoic acid, or a fatty acid, or an alkali metal salt thereof, an aliphatic hydrocarbon such as an n-paraffin, an aromatic hydrocarbon, or for example, a natural organic substance such as peptone, meat extract, fish extract, soybean flour, or bran, singly or in combination thereof, at a concentration of normally 0.01% - 30%, desirably about 0.1% - 20%.

For a culture medium nitrogen source for culturing a microbe, it is possible to use, for example, an inorganic nitrogen compound such as ammonium sulfate, ammonium phosphate, sodium nitrate, or potassium nitrate, a nitrogen-containing organic substance such as urea or uric acid, or a natural organic substance such as peptone, meat extract, fish extract, or soybean flour, singly or in combination thereof, at a concentration of normally 0.01%-20%, desirably about 0.1% - 10%.

Moreover, it is possible to add a phosphate such as potassium dihydrogen phosphate, or a metal salt such as magnesium sulfate, ferrous sulfate, calcium acetate, manganese chloride, copper sulfate, zinc sulfate, cobalt sulfate, or nickel sulfate, as necessary, for growth of a bacteria or improvement of an enzyme activity. A concentration for addition thereof is different depending on a culturing condition, and normally, is 0.01% - 5% for a phosphate, 10 ppm - 1% for a magnesium salt, or about 0.1 ppm - 1,000 ppm for other compounds. Furthermore, for a supply source of a vitamin, an amino acid, a nucleic acid, or the like, it is possible to add, for example, about 1 ppm - 100 ppm of yeast extract, casamino acid, or a yeast nucleic acid, depending on a selected culture medium, for growth of a bacteria or improvement of an enzyme activity.

It is desirable to adjust a pH of a culture medium to 4.5 - 9, desirably 5 - 8. Furthermore, it is useful to fractionate from a culture solution by a method such as centrifugation or membrane filtration, and again suspend and react in water that contains a reaction raw material, physiological saline, a buffer that has a pH adjusted to be comparable to a pH for culturing and composed of phosphoric acid, acetic acid, boric acid, tris(hydroxymethyl)aminomethane, or the like, or a salt thereof, or the like, a microbial or bacterial body that is preliminarily cultured in a culture medium as described previously, in order to reduce an impurity in a reaction fluid and simplify subsequent fractionation of a product. Although a pH during a reaction is normally capable of being retained in a case where a buffer with a sufficient concentration is used, it is desirable to be execute appropriate adjustment by using sodium hydroxide, ammonia, or the like so as to provide a similar pH in a case where deviation from a pH as described above is caused by proceeding of a reaction.

In a case where 1,3-butanediol and/or 1,4-butanediol is stored in a reaction fluid and a reaction rate is lowered thereby, a method is preferable that adds water, physiological saline, a reaction buffer, or the like, into such a reaction fluid depending on a concentration of a product to execute continuous dilution thereof. Furthermore, at a point of time when a reaction rate is lowered, a bacteria is fractionated and a supernatant is recovered as a product solution, wherein a fractionated bacteria is again returned to a solution or suspension that contains a reaction raw material and thereby, it is possible to recover such a reaction rate. It is possible to execute such an operation continuously, or even batchwise, by using a centrifuge, a separation film, or the like.

It is possible to execute separation, recovery, and purification of 1,3-butanediol and/or 1,4-butanediol produced in a reaction fluid by using separation, recovery, and purification means for a general organic compound, after a bacterial body is eliminated from such a reaction fluid by means of centrifugation at a point of time when an amount(s) of produced 1,3-butanediol and/or 1,4-butanediol reach(es) a substantial amount(s), or for such a reaction fluid directly. For example, extraction from a filtrate provided by eliminating a bacterial body and others from a culture solution is executed by using an appropriate organic solvent. 1,3-butanediol and/or 1,4-butanediol is obtained at a high purity by directly executing distillation for such an extract, as well as, further executing extraction with an appropriate solvent again, executing purification that uses chromatography on silica gel or the like, or applying multistep distillation or the like thereto.

### PRACTICAL EXAMPLES

### (Practical examples and Comparative examples)

Next, the present invention will be described in more detail by describing practical examples.

Table 1 illustrates a summary of a relationship between a gene with a sequence number that corresponds to that of a sequence listing and an enzyme that is coded by such a gene.

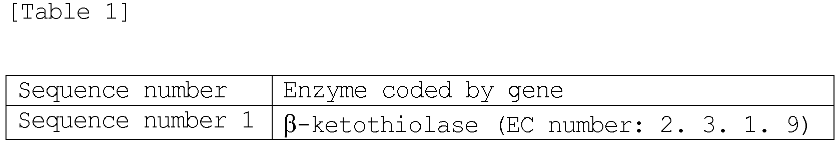

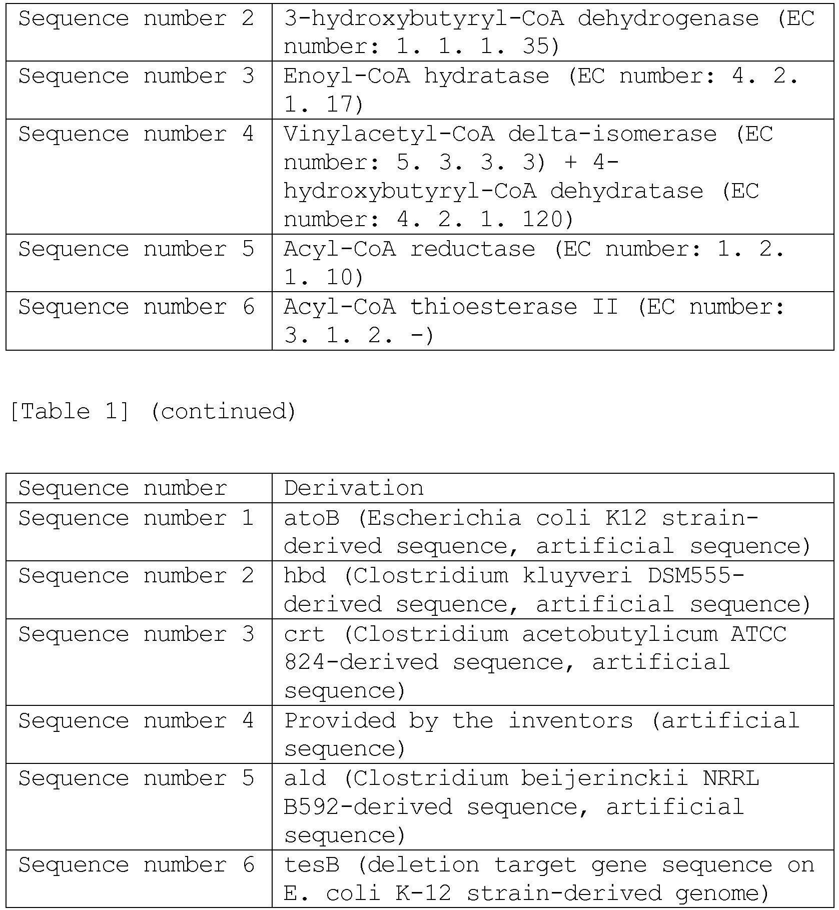

### [Reference Example 1]

JM109 (DE3) ΔTesB strain wherein a CDS region of acyl-CoA thioesterase II gene (tesB) that was an acyl-CoA hydrolase was deleted from a genome of Escherichia coli JM109 (DE3) strain was prepared by using a homologous recombination method with reference to publicly-known entire genome sequence information of Escherichia coli K-12 strain. Here, gene deletion based on a homologous recombination method was executed in accordance with an ordinary method with reference to a non-patent document of Experiments in Molecular Genetics, Cold Spring Harbor Laboratory press (1972); Matsuyama, S. and Mizushima, S., J. Bacteriol., 162, 1196 (1985). A sequence of a deleted CDS region is illustrated in sequence number 6.

A blunt end fragment was prepared by an ordinary method in such a manner that sequences that corresponded to 15 base pairs at an upstream side and a downstream side that contained CAT at an upstream side and ATG at a downstream side of a NdeI site among multi-cloning sites of expression vector pET17b (produced by Novagen, Inc.), respectively, were added to a 5'-end side and a 3'-end side upstream and downstream a gene sequence indicated by sequence number 1, respectively. Ligation of this fragment and a fragment provided by NdeI-treating pET17b (produced by Novagen, Inc.) was executed by In-Fusion HD Cloning Kit (produced by TAKARA BIO INC.) to obtain plasmid pETBD1.

A gene sequence indicated by sequence number 2 was inserted into an NdeI site of pET17b as a target by a method similar to that for pETBD1 to obtain plasmid pETBD2 that contained sequence 2.

An EcoRI site positioned downstream a termination codon of sequence 1 of pETBD1 and derived from multi-cloning sites of pET17b was cleaved by a restriction enzyme treatment to prepare a ring-opened fragment of pETBD1. Then, a fragment was prepared by means of PCR such that sequences that corresponded to 15 bp at an upstream side and 15 bp at a downstream side that contained an EcoRI site of pETBD1 described previously were added upstream and downstream a region of sequence 2 of pETBD2 and a region that contained T7 promoter derived from pET17b upstream thereof. Ligation of two obtained fragments was executed by In-Fusion HD Cloning Kit to obtain plasmid pETBD1-2 that contained sequences 1 and 2.

JM109 (DE3) ΔTesB strain was transformed with plasmid pETBD1-2 in accordance with an ordinary method.

### [Reference Example 2]

A transformed body with plasmid pETBD1-2 was acquired by a method similar to that of Reference Example 1 except that a transformation host was changed to JM109 (DE3).

### [Practical Example 1]

A ring-opened fragment of plasmid pETBD1-2 was prepared by means of inverse PCR wherein a target is a center of GATATC of EcoRV site derived from vector pET17b and positioned downstream of sequence 2 of pETBD1-2 prepared in Reference Example 1.

A gene sequence indicated by sequence number 5 was inserted into an NdeI site of pET17b as a target by a method similar to that for pETBD1 to obtain plasmid pETBD5 that contained sequence 5.

Then, a fragment was prepared by means of PCR such that sequences that corresponded to 15 bp at an upstream side and 15 bp at a downstream side that contained an EcoRV site of pETBD1-2 described previously were added upstream and downstream a region of sequence 5 of pETBD5 and a region that contained T7 promoter derived from pET17b upstream thereof.

Ligation of these obtained fragments was executed by In-Fusion HD Cloning Kit to prepare plasmid pETBD1-2-5 that contained sequences 1, 2, and 5. JM109 (DE3) ΔTesB strain was transformed with such a plasmid in accordance with an ordinary method.

### [Comparative Example 1]

A transformed body with plasmid pETBD1-2-5 was acquired by a method similar to that of Practical Example 1 except that a transformation host was changed to JM109 (DE3).

### [Practical Example 2]

Moreover, while a sequence at a downstream side of sequence 5 of pETBD1-2-5 is a target in accordance with a method similar to that of Practical Example 1, gene sequences indicated by sequence number 3 (that corresponded to enoyl-CoA hydratase) and sequence number 4 (that corresponded to vinylacetyl-CoA delta-isomerase and 4-hydroxybutyryl-CoA dehydratase), together with T7 promoter derived from pET17b, were sequentially added to a downstream side of pETBD1-2-5 prepared in Practical Example 1 by using a method of In-Fusion HD Cloning Kit, to prepare plasmid pETBD1-2-5-3-4. A transformed body of JM109 (DE3) ΔTesB strain with such a plasmid was obtained in accordance with an ordinary method.

### [Comparative Example 2]

A transformed body with plasmid pETBD1-2-5-3-4 was acquired by a method similar to that of Practical Example 2 except that a transformation host was changed to JM109 (DE3).

A transformed body obtained in each of the respective practical examples, comparative examples, and reference examples was aerobically cultured in 5 mL of an LB culture medium that contained 100 mg/L of ampicillin at 37 °C for 12 hours. 0.1 mL of a culture solution was implanted to 5 mL of an LB culture medium that contained 1% of glucose, 100 mg/L of ampicillin, and 0.2 mM of IPTG and aerobically cultured at 30 °C for 48 hours. A supernatant of a culture solution was subjected to high performance liquid chromatography (HPLC: column; Shodex SH-1011 (produced by Showa Denko K. K.), column temperature: 60 °C, eluent: 25 mM sulfuric acid aqueous solution, flow rate: 0.6 mL/min, detection: differential refraction detector). Table 2 illustrates an amount of 3-hydroxybutanoic acid, an amount of 1,3-butanediol, and/or an amount of 1,4-butanediol that were produced in a culture solution.

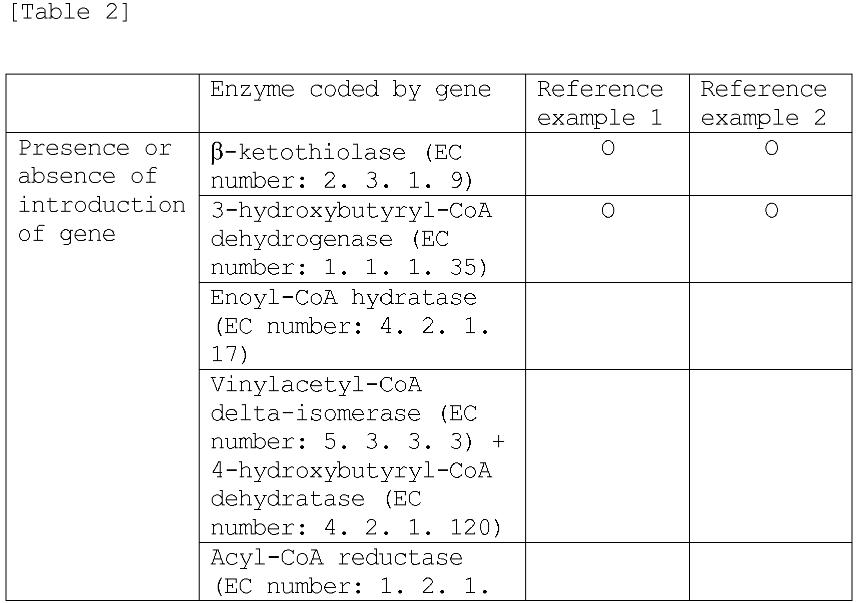

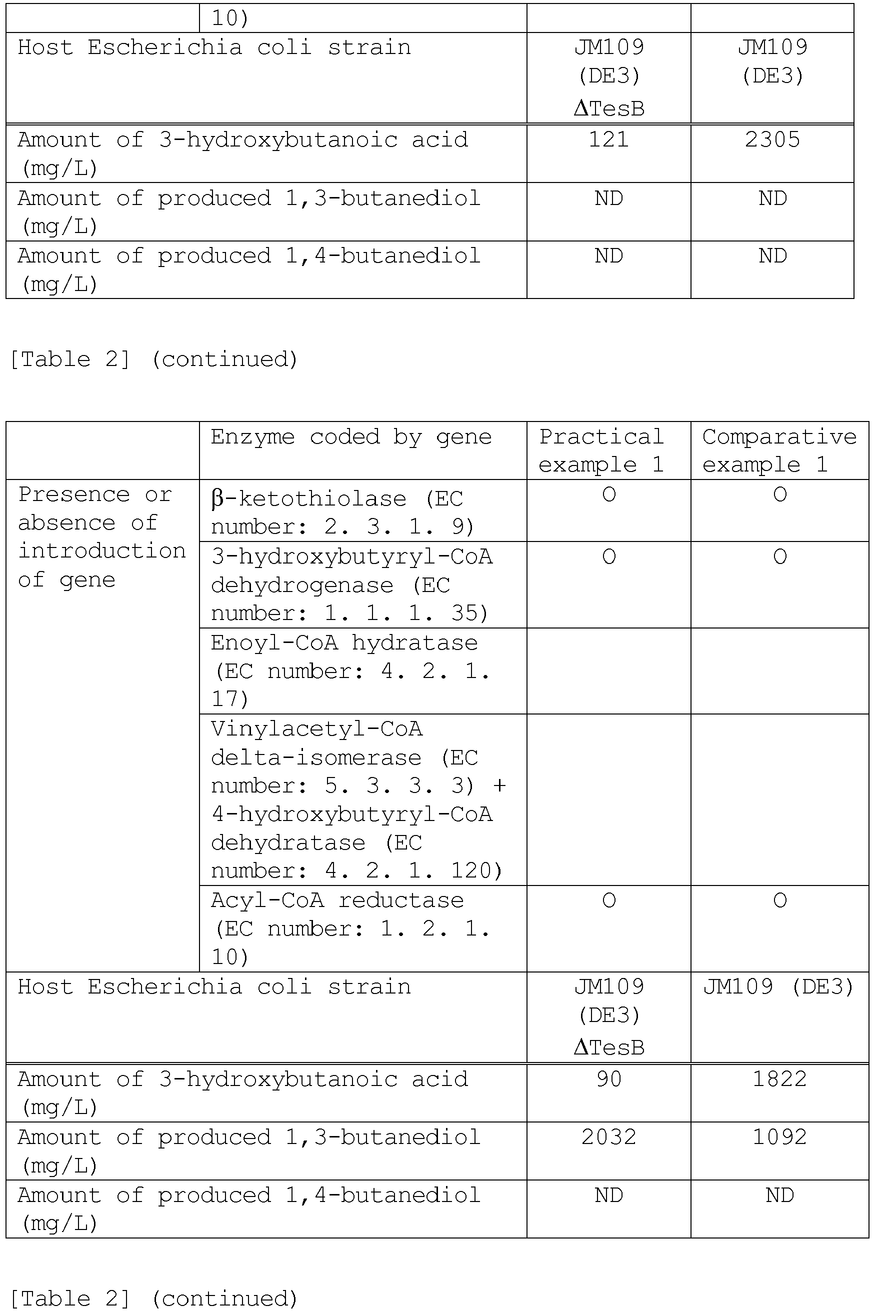

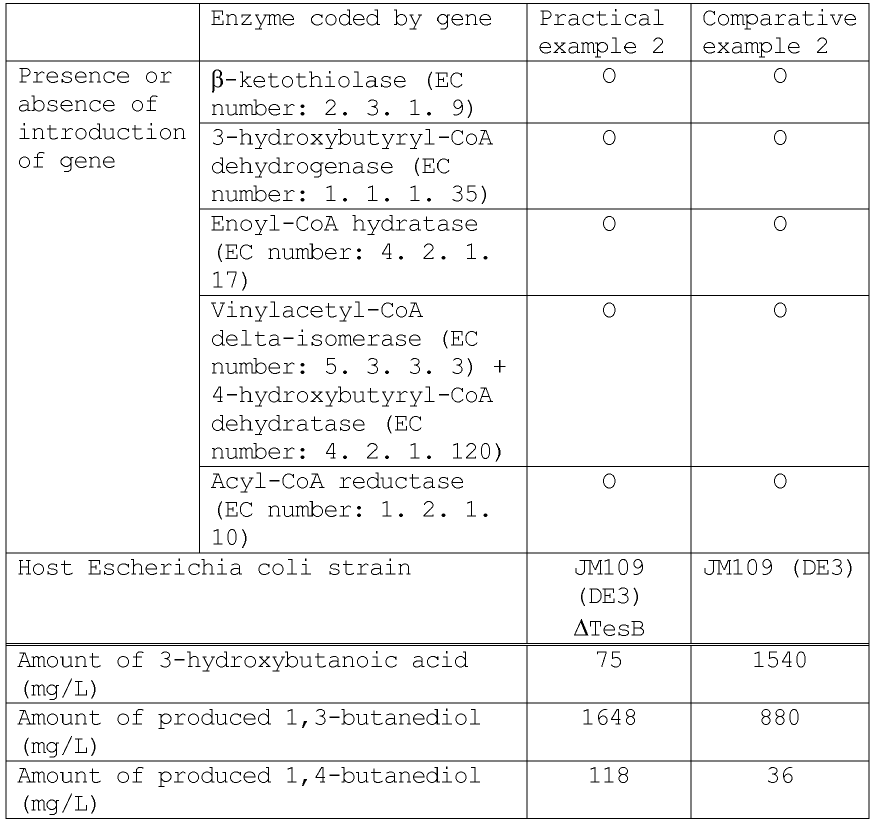

As is clear from Table 2, a manufacturing method for a butanediol that utilizes a biochemical technique according to the present embodiment is such that an activity of a particular acyl-CoA hydratase (EC number: 3. 1. 2. -) that is possessed by a host microbe is deleted or reduced and a hydrolysis reaction from 3-hydroxybutyryl-CoA to 3-hydroxybutanoic acid is suppressed thereby. Accordingly, it is possible to improve productivity of 1,3-butanediol and/or 1,4-butanediol.

The present application claims priority based on Japanese Patent Application No. 2012-271519 filed on December 12, 2012 before the Japan Patent Office and the entire contents of Japanese Patent Application No. 2012-271519 are incorporated by reference in the present application.

## Claims

1. A method of manufacturing a butanediol through 3-hydroxybutyryl-CoA that uses a microbe and/or a culture thereof and utilizes an enzyme reaction that is caused by an acyl-CoA reductase, wherein the microbe in the manufacturing method for a butanediol is **characterized in that** an activity of an acyl-CoA hydratase (EC number: 3. 1. 2. -) is deleted or reduced.

2. The manufacturing method for a butanediol as claimed in claim 1, wherein the acyl-CoA hydratase is an acyl-CoA hydratase classified into EC number: 3. 1. 2. 2 or EC number: 3. 1. 2. 20.

3. The manufacturing method for a butanediol as claimed in claim 1, wherein the microbe includes a gene that codes β-ketothiolase (EC number: 2. 3. 1. 9), a gene that codes 3-hydroxybutyryl-CoA dehydrogenase (EC number: 1. 1. 1. 35), and a gene that codes acyl-CoA reductase (EC number: 1. 2. 1. 10).

4. The manufacturing method for a butanediol as claimed in claim 3, wherein the microbe further includes a gene that codes enoyl-CoA hydratase (EC number: 4. 2. 1. 17), a gene that codes vinylacetyl-CoA delta-isomerase (EC number: 5. 3. 3. 3), and a gene that codes 4-hydroxybutyryl-CoA dehydratase (EC number: 4. 2. 1. 120).

5. The manufacturing method for a butanediol as claimed in claim 1, wherein the microbe is Escherichia coli, a yeast, a coryneform bacteria, or a clostridial bacteria.

6. The manufacturing method for a butanediol as claimed in claim 5, wherein the microbe is Escherichia coli with deleted CDS region of acyl-CoA thioesterase II gene (tesB).

7. A fabrication method for a microbe for manufacturing a butanediol, **characterized by** including a process for deleting or reducing an activity of an acyl-CoA hydratase (EC number: 3. 1. 2. -) of a microbe.

8. The fabrication method for a microbe for manufacturing a butanediol as claimed in claim 7, wherein the acyl-CoA hydratase is an acyl-CoA hydratase classified into EC number: 3. 1. 2. 2 or EC number: 3. 1. 2. 20.

9. A microbe that includes a gene that codes β-ketothiolase (EC number: 2. 3. 1. 9), a gene that codes 3-hydroxybutyryl-CoA dehydrogenase (EC number: 1. 1. 1. 35), and a gene that codes acyl-CoA reductase (EC number: 1. 2. 1. 10) and a deleted or reduced activity of an acyl-CoA hydratase (EC number: 3. 1. 2. -).

10. The microbe as claimed in claim 9, wherein the acyl-CoA hydratase is an acyl-CoA hydratase classified into EC number: 3. 1. 2. 2 or EC number: 3. 1. 2. 20.

11. The microbe as claimed in claim 9, further including a gene that codes enoyl-CoA hydratase (EC number: 4. 2. 1. 17), a gene that codes vinylacetyl-CoA delta-isomerase (EC number: 5. 3. 3. 3), and a gene that codes 4-hydroxybutyryl-CoA dehydratase (EC number: 4. 2. 1. 120).

12. The microbe as claimed in any of claim 9, wherein the microbe is Escherichia coli, a yeast, a coryneform bacteria, or a clostridial bacteria.


SEQUENCE LISTING

<110>  Showa Denko K.K.
<120>  Method of producing butanediols, method of preparing microorganism
for producing butanediols and microorganism
<130>  P20120225(SHD13020PCT)
<160>  6
<170>  PatentIn version 3.5

<210>  1
<211>  1185
<212>  DNA
<213>  Artificial Sequence
<220>
<223>  Escherichia coli atoB gene

<400>  1
atgaaaaatt gtgtcatcgt cagtgcggta cgtactgcta tcggtagttt taacggttca
60

ctcgcttcca ccagcgccat cgacctgggg gcgacagtaa ttaaagccgc cattgaacgt
120

gcaaaaatcg attcacaaca cgttgatgaa gtgattatgg gtaacgtgtt acaagccggg
180

ctggggcaaa atccggcgcg tcaggcactg ttaaaaagcg ggctggcaga aacggtgtgc
240

ggattcacgg tcaataaagt atgtggttcg ggtcttaaaa gtgtggcgct tgccgcccag
300

gccattcagg caggtcaggc gcagagcatt gtggcggggg gtatggaaaa tatgagttta
360

gccccctact tactcgatgc aaaagcacgc tctggttatc gtcttggaga cggacaggtt
420

tatgacgtaa tcctgcgcga tggcctgatg tgcgccaccc atggttatca tatggggatt
480

accgccgaaa acgtggctaa agagtacgga attacccgtg aaatgcagga tgaactggcg
540

ctacattcac agcgtaaagc ggcagccgca attgagtccg gtgcttttac agccgaaatc
600

gtcccggtaa atgttgtcac tcgaaagaaa accttcgtct tcagtcaaga cgaattcccg
660

aaagcgaatt caacggctga agcgttaggt gcattgcgcc cggccttcga taaagcagga
720

acagtcaccg ctgggaacgc gtctggtatt aacgacggtg ctgccgctct ggtgattatg
780

gaagaatctg cggcgctggc agcaggcctt acccccctgg ctcgcattaa aagttatgcc
840

agcggtggcg tgccccccgc attgatgggt atggggccag tacctgccac gcaaaaagcg
900

ttacaactgg cggggctgca actggcggat attgatctca ttgaggctaa tgaagcattt

960

gctgcacagt tccttgccgt tgggaaaaac ctgggctttg attctgagaa agtgaatgtc
1020

aacggcgggg ccatcgcgct cgggcatcct atcggtgcca gtggtgctcg tattctggtc
1080

acactattac atgccatgca ggcacgcgat aaaacgctgg ggctggcaac actgtgcatt
1140

ggcggcggtc agggaattgc gatggtgatt gaacggttga attaa
1185

<210> 2
<211> 849
<212> DNA
<213> Artificial Sequence
<220>
<223> Clostridium kluyveri hbd gene
<400> 2
atgagtatta aaagtgtagc ggttttaggt agtggaacta tgtctcgtgg aattgtgcag
60

gcttttgcag aagcaggtat agatgtaatt atccgtggaa gaactgaagg tagtattgga
120

aaaggtctag cagcagtaaa gaaagcttat gataaaaaag tatcaaaggg gaaaatttcc
180

caggaagatg ctgataaaat agttggaaga gtaagtacaa caactgaact tgaaaaattg
240

gctgattgtg atcttataat agaagcagca tcagaggata tgaatataaa gaaagactat
300

tttggaaaat tagaagaaat atgcaagcct gaaacaattt ttgctactaa tacttcttca
360

ttatctataa ctgaagtagc aacagctaca aagagaccag ataaattcat aggaatgcat
420

ttctttaatc cagcaaatgt tatgaaatta gttgaaatca taagaggtat gaatacttca
480

caagaaactt ttgatattat aaaagaagct tccattaaaa taggaaaaac tcctgtagaa
540

gttgcagaag ctccaggatt tgttgtaaac aagatattag taccaatgat caatgaagca
600

gtaggaattt tggcagaagg aatagcttca gcagaagata tcgatacagc tatgaaatta
660

ggcgctaatc acccaatggg tcctttagca ttaggagatc ttattggact tgatgtagtt
720

cttgcagtta tggatgtact ttatagtgaa actggagatt caaaatatag agctcataca
780

ttacttagaa aatatgtaag agcaggatgg cttggaagaa aatcaggaaa aggattcttc
840

gcttattaa
849

<210>  3
<211>  786
<212>  DNA
<213>  Artificial Sequence
<220>
<223>  Clostridium acetobutylicum crt gene
<400>  3
atggaactaa acaatgtcat ccttgaaaag gaaggtaaag ttgctgtagt taccattaac
60

agacctaaag cattaaatgc gttaaatagt gatacactaa aagaaatgga ttatgttata
120

ggtgaaattg aaaatgatag cgaagtactt gcagtaattt taactggagc aggagaaaaa
180

tcatttgtag caggagcaga tatttctgag atgaaggaaa tgaataccat tgaaggtaga
240

aaattcggga tacttggaaa taaagtgttt agaagattag aacttcttga aaagcctgta
300

atagcagctg ttaatggttt tgctttagga ggcggatgcg aaatagctat gtcttgtgat
360

ataagaatag cttcaagcaa cgcaagattt ggtcaaccag aagtaggtct cggaataaca
420

cctggttttg gtggtacaca aagactttca agattagttg gaatgggcat ggcaaagcag
480

cttatattta ctgcacaaaa tataaaggca gatgaagcat taagaatcgg acttgtaaat
540

aaggtagtag aacctagtga attaatgaat acagcaaaag aaattgcaaa caaaattgtg
600

agcaatgctc cagtagctgt taagttaagc aaacaggcta ttaatagagg aatgcagtgt
660

gatattgata ctgctttagc atttgaatca gaagcatttg gagaatgctt ttcaacagag
720

gatcaaaagg atgcaatgac agctttcata gagaaaagaa aaattgaagg cttcaaaaat
780

agatag
786

<210>  4
<211>  1473
<212>  DNA
<213>  Artificial Sequence
<220>
<223>  putative gene encoding enzyme converting crotonyl-CoA to
       4-hydroxybutyryl-CoA
<400>  4

atgctgatga ccgccgaaca gtacatcgaa agcctgcgca agctgaacac gcgtgtctac
60

atgtttggtg aaaagatcga gaactgggta gaccatccca tgattcgccc ttcgatcaat
120

tgtgtggcaa tgacctatga actggcgcaa gatccccagt atgccgattt gatgaccacg
180

aaatcgaatc tgattggcaa gacgattaat cgcttcgcga acttacacca aagcaccgat
240

gatctgcgca agaaagtcaa aatgcaacgt ctgctgggcc agaaaaccgc gagttgcttt
300

cagcggtgtg ttgggatgga tgcgttcaat gccgtgtttt ccaccactta tgagattgat
360

cagaaatacg ggactaacta ccacaagaac ttcacggagt atctgaaata tattcaagaa
420

aatgacttga tcgtggatgg tgctatgacc gacccgaaag gcgaccgtgg gctggcaccg
480

agtgcccaga aagacccaga tctgtttctg cgcatcgtgg agaaacgcga ggatggtatc
540

gtagtccgtg gtgcgaaagc acatcaaacg ggctcaatta atagccatga gcacatcatt
600

atgccgacca ttgctatgac agaagcggat aaagactatg cggttagttt tgcttgtcca
660

tccgatgcgg atggtttgtt tatgatctat ggtcgccagt cttgcgacac ccgcaaaatg
720

gaggaaggcg cagatattga cttaggcaac aaacagtttg gcggccaaga agcccttgtc
780

gtgtttgaca acgtattcat cccgaatgat cgcattttct tatgccagga atacgatttt
840

gcgggaatga tggtcgaacg cttcgctggt tatcatcgcc agtcttacgg tggctgcaaa
900

gtgggtgtgg gtgacgtcgt tattggagcc gcagcccttg cagcggatta caacggtgcc
960

cagaaggcca gccatgtgaa agacaaactg atcgaaatga cacacttgaa cgaaaccctg
1020

tattgttgtg ggattgcgtg ctcagccgaa ggttatccga ctgcagccgg caattaccag
1080

atcgatctcc tccttgcgaa tgtgtgcaaa cagaacatta cgcgttttcc gtacgagatt
1140

gtacgtctgg cggaagatat tgccggagga ctgatggtta ctatgcctag cgaagcggac
1200

ttcaaaagcg aaacggttgt tgggcgtgat ggcgaaacca ttggcgattt ctgcaacaaa
1260

ttctttgcgg cagcaccgac ttgcaccaca gaagaacgga tgcgtgtgct ccgctttctg
1320

gagaacattt gtttaggcgc atccgctgtt gggtatcgta cagaatcgat gcatggtgcg
1380

ggctctccac aggctcaacg catcatgatt gcccgtcaag gcaatattaa cgcgaagaaa
1440

gaactcgcta aagcgatcgc tggcatcaaa taa
1473

<210> 5
<211> 1407
<212> DNA
<213> Artificial Sequence
<220>
<223> Clostridium beijerinckii ald gene
<400> 5
atgaataaag acacactaat acctacaact aaagatttaa aagtaaaaac aaatggtgaa
60

aacattaatt taaagaacta caaggataat tcttcatgtt tcggagtatt cgaaaatgtt
120

gaaaatgcta taagcagcgc tgtacacgca caaaagatat tatcccttca ttatacaaaa
180

gagcaaagag aaaaaatcat aactgagata agaaaggccg cattacaaaa taaagaggtc
240

ttggctacaa tgattctaga agaaacacat atgggaagat atgaggataa aatattaaaa
300

catgaattgg tagctaaata tactcctggt acagaagatt taactactac tgcttggtca
360

ggtgataatg gtcttacagt tgtagaaatg tctccatatg gtgttatagg tgcaataact
420

ccttctacga atccaactga aactgtaata tgtaatagca taggcatgat agctgctgga
480

aatgctgtag tatttaacgg acacccatgc gctaaaaaat gtgttgcctt tgctgttgaa
540

atgataaata aggcaattat ttcatgtggc ggtcctgaaa atctagtaac aactataaaa
600

aatccaacta tggagtctct agatgcaatt attaagcatc cttcaataaa acttctttgc
660

ggaactgggg gtccaggaat ggtaaaaacc ctcttaaatt ctggtaagaa agctataggt
720

gctggtgctg gaaatccacc agttattgta gatgatactg ctgatataga aaaggctggt
780

aggagcatca ttgaaggctg ttcttttgat aataatttac cttgtattgc agaaaaagaa
840

gtatttgttt ttgagaatgt tgcagatgat ttaatatcta acatgctaaa aaataatgct

900

gtaattataa atgaagatca agtatcaaaa ttaatagatt tagtattaca aaaaaataat
960

gaaactcaag aatactttat aaacaaaaaa tgggtaggaa aagatgcaaa attattctta
1020

gatgaaatag atgttgagtc tccttcaaat gttaaatgca taatctgcga agtaaatgca
1080

aatcatccat ttgttatgac agaactcatg atgccaatat tgccaattgt aagagttaaa
1140

gatatagatg aagctattaa atatgcaaag atagcagaac aaaatagaaa acatagtgcc
1200

tatatttatt ctaaaaatat agacaaccta aatagatttg aaagagaaat agatactact
1260

atttttgtaa agaatgctaa atcttttgct ggtgttggtt atgaagcaga aggatttaca
1320

actttcacta ttgctggatc tactggtgag ggaataacct ctgcaaggaa ttttacaaga
1380

caaagaagat gtgtacttgc cggctaa
1407


<210>  6
<211>  861
<212>  DNA
<213>  Artificial Sequence
<220>
<223>  Escherichia coli
<400>  6
atgagtcagg cgctaaaaaa tttactgaca ttgttaaatc tggaaaaaat tgaggaagga
60

ctctttcgcg gccagagtga agatttaggt ttacgccagg tgtttggcgg ccaggtcgtg
120

ggtcaggcct tgtatgctgc aaaagagacc gtccctgaag agcggctggt acattcgttt
180

cacagctact ttcttcgccc tggcgatagt aagaagccga ttatttatga tgtcgaaacg
240

ctgcgtgacg gtaacagctt cagcgcccgc cgggttgctg ctattcaaaa cggcaaaccg
300

attttttata tgactgcctc tttccaggca ccagaagcgg gtttcgaaca tcaaaaaaca
360

atgccgtccg cgccagcgcc tgatggcctc ccttcggaaa cgcaaatcgc ccaatcgctg
420

gcgcacctgc tgccgccagt gctgaaagat aaattcatct gcgatcgtcc gctggaagtc
480

cgtccggtgg agtttcataa cccactgaaa ggtcacgtcg cagaaccaca tcgtcaggtg
540

tggatccgcg caaatggtag cgtgccggat gacctgcgcg ttcatcagta tctgctcggt
600

tacgcttctg atcttaactt cctgccggta gctctacagc cgcacggcat cggttttctc
660

gaaccgggga ttcagattgc caccattgac cattccatgt ggttccatcg cccgtttaat
720

ttgaatgaat ggctgctgta tagcgtggag agcacctcgg cgtccagcgc acgtggcttt
780

gtgcgcggtg agttttatac ccaagacggc gtactggttg cctcgaccgt tcaggaaggg
840

gtgatgcgta atcacaatta a
861